# EUROPÄISCHE PATENTSCHRIFT

(11) **EP 1 269 927 B1**
(45) Veröffentlichungstag und Bekanntmachung des Hinweises auf die Patenterteilung: **06.04.2011**
(21) Anmeldenummer: 01114960.6
(22) Anmeldetag: 20.06.2001
(51) Int. Cl.: A61B 17/58

(54) **Vergurtungssystem zur Spondylodese der Lendenwirbelsäule**
Connection system for the spinal fusion of the lumbar column
Système de liaison destiné à l'arthrodèse de la région lombaire de la colonne vertébrale

(43) Veröffentlichungstag der Anmeldung: 02.01.2003
(73) Patentinhaber: Copf jun., Franz, D-70178 Stuttgart (DE)
(72) Erfinder: Copf jun., Franz, D-70178 Stuttgart (DE)
(74) Vertreter: Ostertag, Ulrich

(56) Entgegenhaltungen:
- WO-A-94/26190
- DE-A- 19 726 754
- DE-C- 3 625 542
- DE-U- 29 816 374
- US-A- 5 507 211

## Beschreibung

Die vorliegende Erfindung betrifft ein Vergurtungssystem zur Spondylodese der Lendenwirbelsäule nach dem Oberbegriff des Anspruchs 1.

Ein solches Vergurtungssystem ist aus der WO-A-94/26190 bekannt.

Ein vom Markt her bekanntes Vergurtungssystem wird zur Spondylodese der Lendenwirbelsäule, d.h. zur starren Verbindung zweier benachbarter Wirbelkörper z.B. bei beschädigter Bandscheibe, eingesetzt. Es umfaßt zwei Pedikelschrauben, wobei die eine Pedikelschraube im Pedikel des einen Wirbelkörpers und die andere Pedikelschraube im Pedikel des benachbarten Wirbelkörpers verankert werden kann, sowie eine an den beiden Pedikelschrauben befestigbare Verbindungsstange. Vor dem Einsetzen der Verbindungsstange kann an den Pedikelschrauben ein Repositionsinstrument befestigt werden. Durch entsprechende Einstellung des Repositionsinstruments kann sowohl die Relativposition der Wirbelkörper in ventrodorsaler Richtung als auch der Lordosewinkel eingestellt werden.

Nachteilig bei diesem Vergurtungssystem ist jedoch, daß für seinen Einsatz die paravertebrale Muskulatur über eine Strecke von 15 bis 20 cm von der Wirbelsäule freipräpariert werden muß, und zwar über die Gelenkfortsätze lateral hinaus. Dabei wird die neurale und die vaskuläre Versorgung in diesem Bereich weitgehend unterbrochen. Die relativ große Wunde bleibt über einen Zeitraum von ca. eineinhalb bis zweieinhalb Stunden offen, was zu einem entsprechenden Infektionsrisiko führt. Zuzüglich muß bei einer solch großen Wunde mit einem Blutverlust von ca. 300 bis 500 ml gerechnet werden, was für den Kreislauf des Patienten belastend ist.

Aus der eingangs genannten DE 197 26 754 A1 ist ein weiteres Vergurtungssystem bekannt, mit welchem im Bereich der Lendenwirbelsäule ebenfalls eine Versteifung vorgenommen werden kann. Dieses Vergurtungssystem wird jedoch nicht in den Pedikeln der Wirbel, sondern in deren Spongiosa verankert. Hierzu wird eine andere Operationsweise verwendet, bei welcher der Zugang zur Lendenwirbelsäule von der Seite des Patienten her gewonnen wird. Bei diesem Operationsverfahren ist zwar eine Dilatation der Wirbelsäule selbst, nicht aber eine Reposition der Wirbel in vetro-dorsaler Richtung möglich.

Ein Vergurtungssystem, welches Pedikelschrauben verwendet, geht aus der DE 36 25 542 C1 hervor. Die Pedikelschrauben sind zunächst sehr lang; mittels einer daran extrakorporal befestigten Gewindespindel ist eine ventro-dorsale Reposition der Wirbel möglich, nach deren Beendigung und Fixierung die Gewindespindel entfernt wird und überstehende Teile der Pedikelschrauben abgetrennt werden.

Aus der WO 94/26190 A1 ist eine Pedikelschraube mit einer Befestigungseinrichung für einen Längsträger bekannt; ein Werkzeug zum Einschrauben von Pedikelschrauben ist der US 5 507 211 zu entnehmen.

Die vorliegende Erfindung hat daher die Aufgabe, ein Vergurtungssystem der eingangs genannten Art so weiterzubilden, dass die Repositionierung zweier benachbarter Wirbelkörper und deren Vergurtung auf für den Patienten schonende und weitgehend risikolose Art und Weise durchgeführt werden kann.

Diese Aufgabe wird durch ein Vergurtungssystem mit den im Anspruch 1 angegebenen Merkmalen gelöst.

Das erfindungsgemäße Vergurtungssystem ermöglicht es, das Repositionsinstrument relativ weit weg von den Wirbelkörpern einzusetzen. Dies wird dadurch ermöglicht, daß an den Verankerungselementen jeweils ein Verlängerungselement lösbar befestigt werden kann. Das Repositionsinstrument wird wiederum an diesen Verlängerungselementen angebracht. So wird es möglich, die Verankerungselemente, in den meisten Fällen wohl Pedikelschrauben, zunächst durch einen mikrochirurgischen Eingriff in den Pedikeln zu verankern. Dabei werden die paravertebralen Muskeln einfach durchstoßen. Anschließend werden, ebenfalls mikrochirurgisch, die Verlängerungselemente an den Verankerungselementen befestigt und durch die kleinen Öffnungen in der Haut nach außen geführt. Das Repositionsinstrument kann jetzt über der Haut des Patienten angesetzt werden, so daß eine große Wunde mit den eingangs genannten Nachteilen nicht mehr erforderlich ist. Vorteilhafte Weiterbildungen der Erfindung sind in Unteransprüchen angegeben.

Gemäß der Weiterbildung nach Anspruch 2 ist das Verlängerungselement an dem Verankerungselement axial fluchtend befestigbar. Dies erleichtert die Handhabung des Verlängerungselements und die Ausrichtung der Wirbelkörper.

Das Aufschrauben und das Entfernen des Verlängerungselements wird durch die Weiterbildung gemäß Anspruch 3 erleichtert, wonach das Verlängerungselement einen Angriffsabschnitt für ein Werkzeug aufweist.

Wie bereits eingangs ausgeführt wurde, soll die Relativposition der beiden Wirbelkörper nach erfolgter Repositionierung durch eine an den Verankerungselementen mit Hilfe der Verbindungseinrichtungen befestigbaren Verbindungsstange dauerhaft festgelegt werden. Damit auch das Einsetzen dieser Verbindungseinrichtungen innerhalb des mikrochirurgischen Eingriffes möglich ist, wird in Anspruch 4 vorgeschlagen, daß die jeweilige Verbindungseinrichtung ein mit dem Verankerungselement lösbar verbindbares Befestigungsteil mit einem zumindest bereichsweise kugelpfannenförmigen Lagerabschnitt, ein zweigeteiltes Lagerelement für die Verbindungsstange mit einem mindestens bereichsweise kugelig gekrümmten, zu dem kugelpfannenförmigen Lagerabschnitt komplementären Lagerabschnitt und ein Klemmelement umfaßt, welches die Verbindungsstange, das Befestigungsteil und das Lagerelement in Einbaulage beaufschlagt und hierdurch festlegt.

Durch diese Weiterbildung der Erfindung wird eine kugelkopfartige Verbindungseinrichtung geschaffen, deren Einzelteile nacheinander durch die vorhandene kleine Hautöffnung auf dem Verankerungselement aufgebaut und schließlich aufgrund des Klemmelements dauerhaft verriegelt werden kann.

Damit die Verbindung der beiden Wirbelkörper möglichst starr ist, wird gemäß Anspruch 5 vorgeschlagen, daß der kugelig gekrümmte Abschnitt und der komplementäre kugelpfannenförmige Abschnitt Oberflächen mit einem hohem Reibungswert aufweist, derart, daß sie sich unter Pressung nicht gegeneinander verschieben können.

Eine alternative Verbindungseinrichtung ist in Anspruch 6 vorgeschlagen: Bei diesem weist die Verbindungseinrichtung ein mit dem Verankerungselement in axialer Richtung verbindbares Befestigungsteil auf, das einen seitlichen Lagerabschnitt besitzt, in dem ein Endabschnitt der Verbindungsstange fest aufgenommen werden kann. Diese Verbindungseinrichtung ist leicht realisierbar und auch unter beengten Verhältnissen im Operationsgebiet sicher einsetzbar.

In den Ansprüchen 7 bis 14 sind vorteilhafte Weiterbildungen des in Anspruch 6 angegebenen Vergurtungssystems beansprucht.

Es ist grundsätzlich wichtig, daß das Verlängerungselement vom Verankerungselement gelöst werden kann, ohne daß dessen Verankerung mit dem Knochen gelöst wird. Ist das Verlängerungselement auf dem Verankerungselement durch eine Drehbewegung befestigbar, ist ein Gegenhalter hilfreich, der bei aufgesetztem Verlängerungselement mit dem Verankerungselement drehfest und lösbar verbunden werden kann.

Eine einfache drehfeste und doch lösbare Verbindung des Gegenhalters mit dem Verankerungselement ist möglich, wenn der Gegenhalter Ausnehmungen oder Vorsprünge aufweist, die in hierzu komplementäre Ausnehmungen des Verankerungselements eingreifen können.

Nachfolgend werden Ausführungsbeispiele der Erfindung unter Bezugnahme auf die Zeichnung im Detail erläutert. In dieser zeigen:
- Figur 1:: eine Explosionsdarstellung im Längsschnitt einzel- ner Elemente eines Vergurtungssystems, eines Repositionsinstruments und eines Gegenhalters;
- Figur 2:: eine perspektivische Explosionsdarstellung der Pe- dikelschraube, des Verlängerungselements und des Gegenhalters von Figur 1;

- Figur 3:: eine Seitenansicht eines ersten Ausführungsbei- spiels einer Verbindungseinrichtung mit einer Verbindungsstange;
- Figur 4:: eine Vorderansicht der Verbindungseinrichtung von Figur 3;
- Figur 5:: einen Schnitt durch die Verbindungseinrichtung von Fig. 3 längs der linie V-V;
- Figur 6:: einen Schnitt durch die Verbindungseinrichtung von Figur 3 längs der Linie VI-VI;

Die folgenden Figuren sind nicht Gegenstand der bean spruchten Erfindung. Es zeigen:
- Figur 7:: einen Längsschnitt durch ein weiteres Ausführungs- beispiel einer Verbindungseinrichtung zu einem ersten Zeitpunkt des Zusammenbaus;
- Figur 8:: einen Längsschnitt durch die Verbindungseinrich- tung von Figur 7 zu einem zweiten Zeitpunkt während des Zusammenbaus; und
- Figur 9:: eine grobschematische Darstellung des Operations- gebiets von außen gesehen.

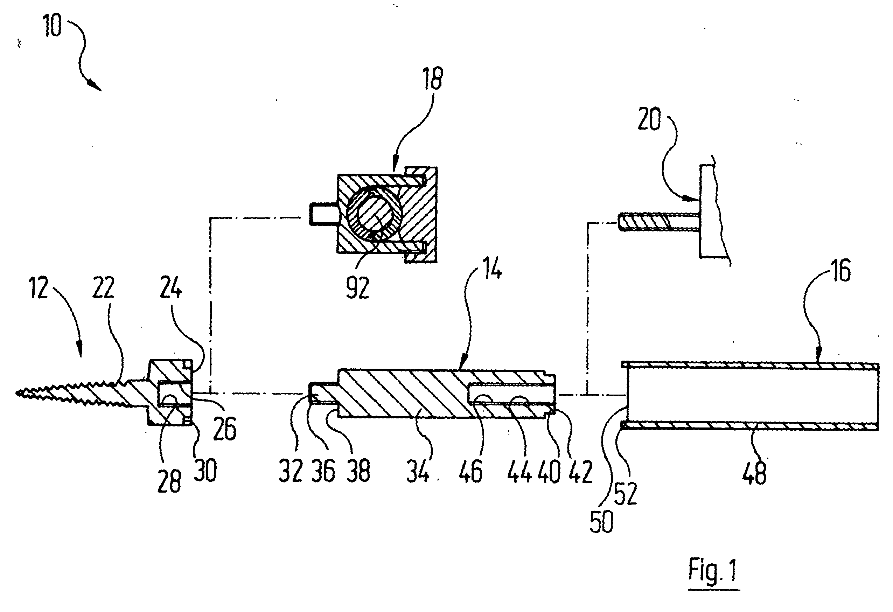

Ein Vergurtungssystem trägt in Figur 1 insgesamt das Bezugszeichen 10. Es umfaßt zwei als Pedikelschrauben ausgebildete Verankerungselemente 12, von denen eine in der Zeichnung dargestellt ist, eine Verbindungsstange 92 und für jede Pedikelschraube 12 ein Verlängerungselement 14 und eine Verbindungseinrichtung 18. Ferner sind in Figur 1 ein Gegenhalter 16 und teilweise ein Repositionsinstrument 20 dargestellt.

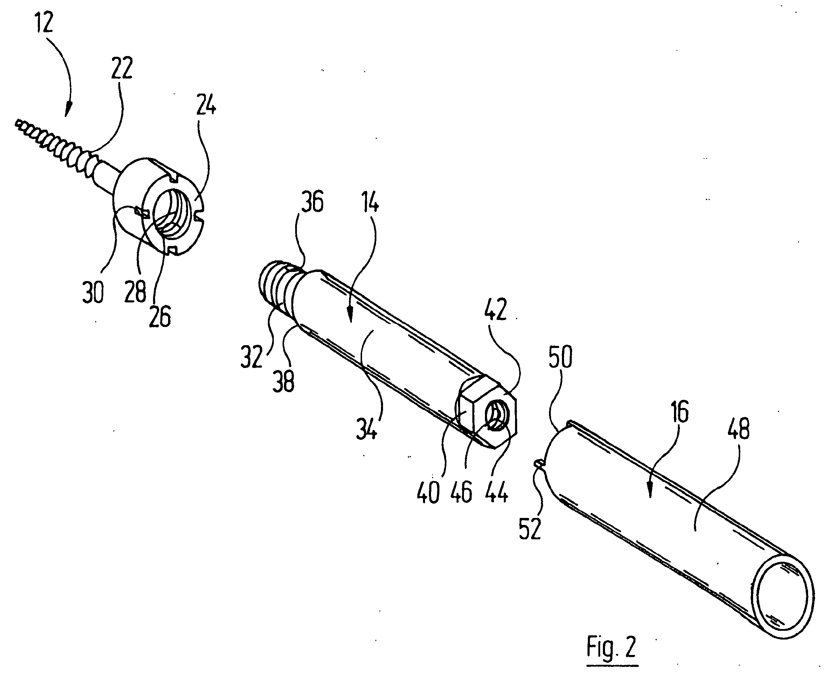

Die Pedikelschraube 12 hat einen zylindrischen Kopf 13 und einen konischen Schraubabschnitt 15, der auf seiner äußeren Mantelfläche ein Gewinde 22 trägt (vergleiche auch Figur 2). Der Schraubabschnitt 15 hat in etwa die geometrische Ausbildung einer Holzschraube. In die in den Figuren 1 und 2 rechte Stirnfläche 24 des Kopfs 13 der Pedikelschraube 12 ist eine mittige axiale Gewindebohrung 26 eingebracht. Am radial äußeren Rand der Stirnfläche 24 des Kopfs 13 sind über den Umfang verteilt vier Ausnehmungen 30 vorhanden, auf deren Bedeutung weiter unten im Zusammenhang mit dem Gegenhalter 16 eingegangen wird.

Das Verlängerungselement 14 weist an seinem der Pedikelschraube 12 zugewandten Ende einen Gewindezapfen 32 sowie einen an diesen angeformten länglichen zylindrischen Stangenabschnitt 34 auf. Der Gewindezapfen 32 ist so ausgebildet, daß er in die Gewindebohrung 26 im Kopf 13 der Pedikelschraube 12 eingeschraubt werden kann. Der Stangenabschnitt 34 hat einen etwas größeren Durchmesser als der Gewindezapfen 32, so daß zwischen diesen beiden ein Absatz 38 gebildet ist. Der Durchmesser des Stangenabschnittes 34 ist etwas kleiner als der Durchmesser des Kopfs 13 der Pedikelschraube 12 und entspricht in etwa dem Abstand der radial inneren Ränder gegenüberliegender Ausnehmungen 30 in der Stirnfläche 24 des Kopfs 13 der Pedikelschraube 12.

Das von der Pedikelschraube 12 abgelegene Ende des Stangenabschnitts 34 ist als Mehrkantabschnitt 40 für einen Schraubenschlüssel ausgebildet. In die Stirnfläche 42 des Mehrkantabschnitts 40 ist eine axiale Gewindebohrung 44 eingebracht, welche, wie weiter unten noch im Detail erläutert ist, eine Befestigungseinrichtung für das Repositionsinstrument 20 bildet.

Der Gegenhalter 16 besteht aus einem Rohr 48, dessen Innendurchmesser so gewählt ist, daß es unter leichtem Spiel über den Stangenabschnitt 34 des Verlängerungselements 14 geschoben werden kann. An seinem in den Figuren 1 und 2 in Richtung zur Pedikelschraube 12 zeigenden Rand 50 sind über den Umfang verteilt vier Vorsprünge 52 angeformt, die so plaziert und bemessen sind, daß sie in die Ausnehmungen 30 in der Stirnfläche 24 des Kopfs 13 der Pedikelschraube 12 eingreifen können. Die Länge des Rohres 48 ist etwas kürzer als die des Stangenabschnitts 34. Wenn das Verlängerungselement 14 auf die Pedikelschraube 12 aufgeschraubt und der Gegenhalter 16 über das Verlängerungselement 14 bis zur Pedikelschraube 12 geschoben ist, ist somit der Mehrkantabschnitt 40 nicht von dem Rohr 48 bedeckt.

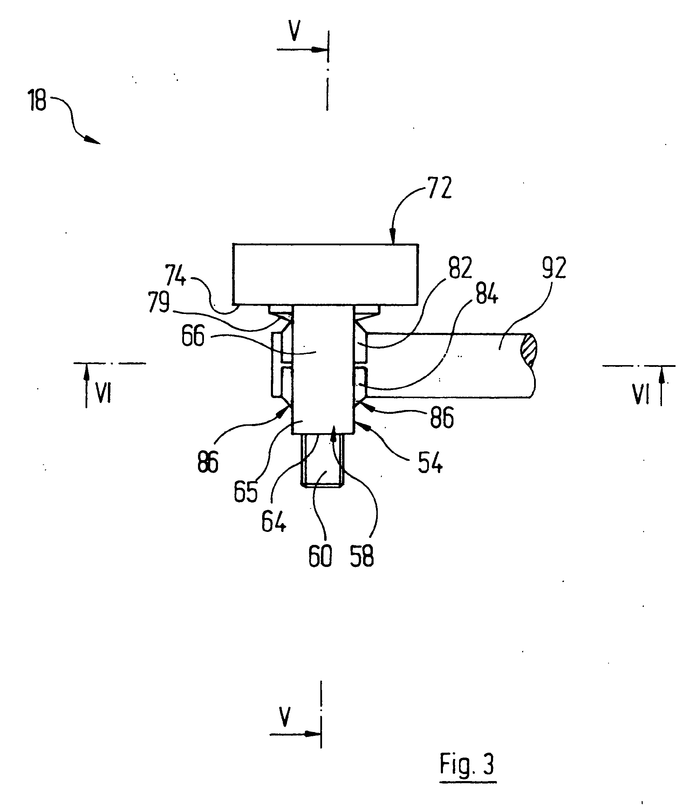

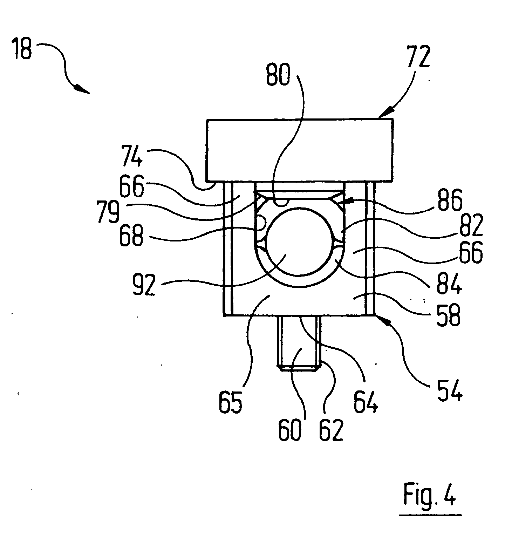

Nun wird auf die Figuren 3 bis 6 Bezug genommen, in denen ein erstes Ausführungsbeispiel einer Verbindungseinrichtung 18 dargestellt ist. Diese dient dazu, die Verbindungsstange 92 an der Pedikelschraube 12 starr zu befestigen. Dies wird im Detail weiter unten noch erläutert werden. Die Verbindungseinrichtung 18 umfaßt ein Befestigungsteil 54, ein Klemmelement 72 und ein Lagerelement 86 für die Verbindungsstange 92.

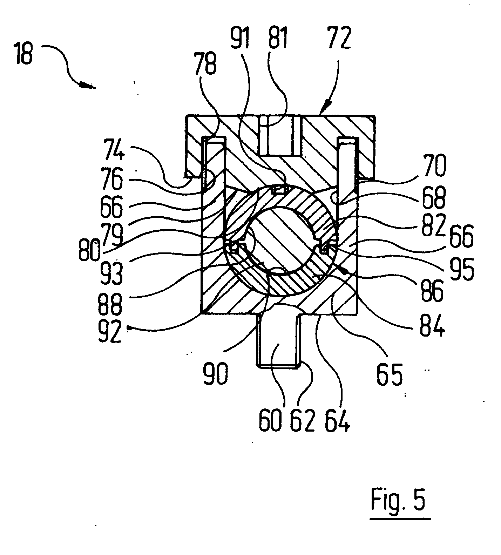

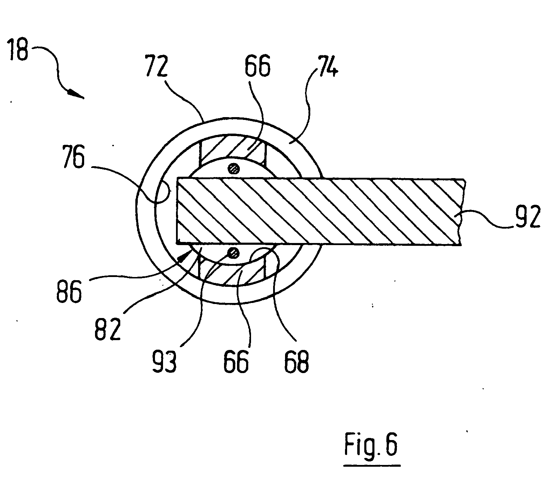

Das Befestigungsteil 54 weist einen Gewindezapfen 60 und einen Lagerabschnitt 58 auf. Der Gewindezapfen 60 ist gleich ausgebildet wie der Gewindezapfen 32 des Verlängerungselements 14, er ist also ebenfalls in die Gewindebohrung 26 im Kopf 13 der Pedikelschraube 12 einschraubbar. Während der Gewindezapfen 60 im wesentlichen zylindrische Form hat, hat der Lagerabschnitt 58 in der in Figur 5 dargestellten Schnittansicht die Außenkontur eines Rechtecks, welches, wie aus den Figuren 3 und 6 ersichtlich ist, nur eine geringe Dicke aufweist. Zwischen Gewindezapfen 60 und Lagerabschnitt 58 ist somit ein Absatz 64 vorhanden.

Der Lagerabschnitt 58 umfaßt einen zum Gewindezapfen 60 transversalen Basisabschnitt 65 und zwei sich von den Enden des Basisabschnitts parallel zur Achse des Gewindezapfens 60 in entgegengesetzter Richtung zum Gewindezapfen 60 erstreckende längliche Seitenabschnitte 66. Der Basisabschnitt 65 und die Seitenabschnitte 66 begrenzen somit eine U-förmige Ausnehmung 68 (Figur 5). Die Wand der Ausnehmung 68 ist senkrecht zur Schnittebene von Figur 5 gekrümmt, und zwar derart, daß die Ausnehmung 68 in ihrem unteren Bereich eine insgesamt halbkugelpfannenförmige und im seitlichen Breich zylindrische Kontur hat. Die Außenwände der Seitenabschnitte 66 sind ebenfalls senkrecht zur Schnittebene von Figur 5 gekrümmt, und zwar derart, daß sie auf einem gemeinsamen Zylindermantel liegen. In ihrem oberen Bereich tragen die Außenwände der Seitenabschnitte 66 jeweils Gewinde 70, deren Windungen aufeinander abgestimmt sind, so daß ein Außengewinde auf noch darzustellende Art und Weise in sie eingreifen kann.

Beim Klemmelement 72 handelt es sich im wesentlichen um eine kreiszylindrische Scheibe, deren Außendurchmesser größer ist als der Außendurchmesser der Seitenwände 66. In die in Einbaulage dem Befestigungsteil 54 zugewandte Stirnseite 74 des Klemmelements ist eine Ringnut 76 eingebracht, deren Außendurchmesser dem Durchmesser des Zylindermantels entspricht, auf dem die Seitenabschnitte 66 des Befestigungsteils 54 liegen, und deren Innendurchmesser dem Durchmesser der Ausnehmung 68 zwischen dem Basisabschnitt 65 und den Seitenabschnitten 66 des Lagerabschnitts 58 des Befestigungsteils 54 entspricht. In der radial äußeren Wand der Ringnut 76 ist ein Gewinde 78 vorhanden, welches mit den Gewinden 70 auf den Außenwänden der Seitenabschnitte 66 des Befestigungsteils 54 zusammenarbeiten kann.

Der innerhalb der Ringnut 76 liegende Bereich der Stirnseite 74 des Klemmelements 72 steht axial etwas über den außerhalb der Ringnut 76 liegenden Bereich über. Er weist einen radial äußeren konischen Abschnitt 79 auf, welcher in eine zentrische kugelige Ausnehmung 80 mündet. Der Krümmungsradius der Ausnehmung 80 entspricht dem Krümmungsradius der Ausnehmung 68 im Befestigungsteil 54.

In die in Einbaulage vom Befestigungsteil 54 abgelegene Stirnseite des Klemmelements 72 ist eine zentrische Sechskantausnehmung 81 eingebracht.

Das Lagerelement 86 besteht aus zwei Hälften 82 und 84. Diese haben jeweils halbkugelförmige Außenkontur und weisen in den einander zugewandten Stirnflächen Ausnehmungen 88 bzw. 90 mit zylindrisch gekrümmter Mantelfläche auf. Der Durchmesser des in Einbaulage insgesamt eine kugelförmige Außenkontur aufweisenden Lagerelements 86 ist so gewählt, daß dieses zu der kugelpfannenförmigen Ausnehmung 68 im Lagerabschnitt 58 des Befestigungsteils 54 und zu der kugeligen Ausnehmung 80 im Klemmelement 72 komplementär ist.

An die Stirnflächen der oberen Hälfte 82 des Lagerelements 86 sind zwei Zapfen 93 angeformt, die in Einbaulage in Ausnehmungen 95 in den Stirnflächen der unteren Hälfte 84 unter relativ engem und reibungsbehaftetem Gleitspiel eingreifen.

Der Durchmesser der Ausnehmungen 88 und 90 des Lagerelements 86 entspricht dem Durchmesser der Verbindungsstange 92. Die Tiefe der Ausnehmungen 88 und 90 in den beiden Hälften 82 und 84 des Lagerelements 86 ist dabei kleiner als ihr Radius, so daß zwischen den beiden Hälften 82 und 84 ein geringer Spalt verbleibt, wenn die Verbindungsstange 92 in die beiden halbzylindrischen Ausnehmungen 88 und 90 eingelegt ist. Hierdurch ist es möglich, die beiden Hälften 82 und 84 formschlüssig unter hoher Pressung auf die Verbindungsstange 92 aufzudrücken.

Die äußeren Mantelflächen der beiden Hälften 82 und 84 des Lagerelements 86 und die mit diesen in Einbaulage zusammenarbeitenden Mantelflächen der Ausnehmungen 68 und 80 im Befestigungsteil 54 und im Klemmelement 72 sind relativ rauh, so daß sie in Einbaulage unter hoher Reibung zusammenarbeiten. Gleiches gilt für die Oberfläche der Verbindungsstange 92 und die halbzylindrischen Ausnehmungen 88 und 90 im Lagerelement 86.

In der oberen Hälfte 82 des Lagerelements 86 ist ferner eine zentrische Gewindebohrung 91 vorhanden.

Das oben beschriebene Vergurtungssystem 10 und das Repositionsinstrument 18 sowie der Gegenhalter 16 können folgendermaßen eingesetzt werden:

Zunächst wird in einem mikrochirurgischen Eingriff eine Hautinzision von ca. 4cm vorgenommen und der Processus spinosus der beiden betroffenen Wirbelkörper dargestellt. Die paravertrebrale Muskulatur wird durchstoßen und der Bogen, der mediale Anteil der Facette und das Ligamentum flavum werden dargestellt. In einem hier nicht näher erläuterten Verfahren werden anschließend die genauen Positionen der einzubringenden Pedikelschrauben 12 festgelegt und markiert sowie ein Zugang zu den Pedikeln der Wirbelkörper geschaffen. Ferner wird eine Bohrung durch den Pedikel durchgeführt und ein Gewinde in diese Bohrung eingeschnitten. Dann wird das Verlängerungselement 14 mit seinem Gewindezapfen 32 in die Gewindebohrung 26 im Kopf 13 der Pedikelschraube 12 eingeschraubt, bis der Absatz 38 an dessen Stirnfläche 24 des Kopfs 13 anliegt. Nun kann die Pedikelschraube 12 bequem am Verlängerungselement 14 gehalten und durch die kleine Hautöffnung und die paravertebrale Muskulatur hindurch in den Pedikel eingedreht werden. Die Länge des Verlängerungselements 14 ist so bemessen, daß dieses nach Eindrehen der Pedikelschraube 12 noch etwas über die Haut des Patienten nach außen vorsteht. Dieser Vorgang wird an allen notwendigen Pedikelstellen wiederholt, für zwei benachbarte Wirbelkörper normalerweise also an vier Stellen.

Nun wird über die Gewindebohrungen 44 in den Stangenabschnitten 34 der Verlängerungselemente 14 das Repositionsinstrument 20 befestigt, mit dem sowohl die Relativstellung der betroffenen Wirbelkörper in ventrodorsaler Richtung als auch der Lordosewinkel eingestellt werden kann.

Nach erfolgter Reposition wird das Repositionsinstrument 20 von den Verlängerungselementen 14 entfernt. Bereits hierbei ist der Mehrkantabschnitt 40 am Verlängerungselement 14 hilfreich, da er beim Abschrauben des Repositionsinstruments 20 ein Festhalten des Verlängerungselements 14 z.B. mit einem Schraubenschlüssel ermöglicht. Nun werden die Verlängerungselemente 14 von den Pedikelschrauben 12 gelöst, indem zunächst das Rohr 48 des Gegenhalters 16 mit den Vorsprüngen 52 voraus über das jeweilige Verlängerungselement 14 geschoben wird, bis seine Vorsprünge 52 in die Ausnehmungen 30 in der Stirnfläche 24 des Kopfs 13 der entsprechenden Pedikelschraube 12 eingreifen. Der Operateur hält nun den Gegenhalter 16 fest, setzt mit einem Schraubenschlüssel an dem aus dem Rohr 48 des Gegenhalters 16 hervorstehenden Mehrkantabschnitt 40 des Verlängerungselements 14 an und schraubt das Verlängerungselement 14 los. Durch den Gegenhalter 16 ist dabei sichergestellt, daß sich beim Abschrauben des Verlängerungselements 14 die Pedikelschraube 12 nicht aus dem Pedikel löst.

Nun verbindet der Operateur die Pedikel von Pedikelpaaren benachbarter Wirbelkörper mit Hilfe der Verbindungsstange 92 und der Verbindungseinrichtungen 18.

Zunächst schraubt der Operateur das Befestigungsteil 54 mit dem Gewindezapfen 60 in die Gewindebohrung 26 im Kopf 13 der Pedikelschraube 12 des ersten Pedikels eines Pedikelpaares ein, bis der Absatz 64 zwischen dem Gewindezapfen 60 und dem Lagerabschnitt 58 des Befestigungsteils 54 an der Stirnfläche 24 des Kopfs 13 der Pedikelschraube 12 in Anlage kommt. Sollte die Ausrichtung des Lagerabschnitts 58 nicht den Erfordernissen entsprechen, kann der Operateur das Befestigungsteil 54 nochmals lösen und ein anderes Befestigungsteil einschrauben, dessen Gewindezapfen 60 gegenüber dem Gewindezapfen 60 des vorhergehenden Befestigungsteils 54 etwas winkelversetzt ist. Dies wiederholt der Operateur an der anderen Pedikelschraube 12 am zweiten Pedikel eines Pedikelpaares.

Nun gibt es zwei Möglichkeiten für das weitere Vorgehen:

Bei der ersten Variante ist an dem einen Ende der Verbindungsstange 92 bereits ein Lagerelement 86 fest angebracht und auf das andere Ende ein Lagerelement 86 aufgeschoben. Dies geschieht dadurch, daß die Verbindungsstange 92 zwischen der unteren Hälfte 84 und der oberen Hälfte 82 des Lagerelements verklemmt wird, was aufgrund des Reibschlusses der Zapfen 93 der obere Hälfte 82 in den Ausnehmungen 95 in der unteren Hälfte 84 möglich ist.

Jetzt setzt der Operateur das angeformte Lagerelement 86 in die Ausnehmung 68 im Befestigungsteil 54 am ersten Pedikel eines Pedikelpaares, führt das Klemmelement 72 an einer in der Sechskantausnehmung 81 eingreifenden Stange (nicht dargestellt) ein und schraubt es auf die Seitenabschnitte 66 des Befestigungsteils 54 auf, ohne es ganz festzuziehen. Dabei kommt die kugelige Ausnehmung 80 in Anlage an die Oberfläche des Lagerelements 86, derart, daß das Lagerelement 86 in der Ausnehmung 68 zwar gesichert, aber noch beweglich ist.

Das nur auf die Verbindungsstange 92 aufgeschobene Lagerelement 86 wird nun axial auf die Verbindungsstange 92 so bewegt und die Verbindungsstange 92 ggf. so gebogen, bis das Lagerelement 86 in die Ausnehmung 68 im Befestigungselement 54 am entsprechenden zweiten Pedikel eines Pedikelpaares eingesetzt werden kann. Aufgrund der in einem weiten Winkelbereich kardanischen Lagerung der Verbindungsstange 92 über das zwischen dem Befestigungsteil 54 und dem Klemmelement 72 am ersten Pedikel gehaltene Lagerelement 86 kann der Operateur dabei die Orientierung der Verbindungsstange 92 den Erfordernissen des Operationsgebiets anpassen. Dieser Winkelbereich wird auch dadurch gewährleistet, daß der radial außerhalb der Ringnut 76 liegende Bereich der Stirnfläche 74 des Klemmelements 72 gegenüber dem radial innenliegenden Bereich 79 etwas zurückspringt.

Jetzt wird das entsprechende Klemmelement 72 wie beim ersten Pedikel eingeführt und auf die Seitenabschnitte 66 des Befestigungsteils 54 aufgeschraubt. In der gewünschten Endlage der Verbindungsstange 92 werden beide Klemmelemente 72 festgezogen.

Da die Tiefe der Ausnehmungen 88 und 90 in den beiden Hälften 82 und 84 des Lagerelements 86 etwas kleiner ist als der Radius der Verbindungsstange 92, ist auch bei maximal angezogenem Klemmelement 72 zwischen den beiden Hälften 84 und 82 des Lagerelements 86 noch ein geringer Spalt vorhanden. Hierdurch wird gewährleistet, daß zwischen der Verbindungsstange 92 und der Oberfläche der Ausnehmungen 88 und 90 im Lagerelement 86 eine zuverlässige Pressung vorliegt, durch die, zusammen mit der aufgrund der hohen Rauhigkeit der aneinanderliegenden Oberflächen vorliegenden Reibung, die Verbindungsstange 92 letztlich gegenüber der jeweiligen Pedikelschraube 12 starr gehalten ist.

Die Verbindungseinrichtung 18 ermöglicht es also, daß der Operateur die dorsale Zugurtung über einen kleinen chirurgischen Zugang vornimmt, welcher im wesentlichen in Längsrichtung der Pedikelschraube 12 liegt. Ein lateraler Zugang und somit eine Vergrößerung der Wunde ist nicht erforderlich.

Bei der zweiten Variante sind die unteren Hälften 84 der Lagerelemente 86 mit einem kleinen Haftmitteltropfen (nicht dargestellt) in die Ausnehmungen 68 in den Lagerabschnitten 58 der Befestigungsteile 54 eingeklebt. Die Befestigungsteile 54 werden also gemeinsam mit den Hälften 84 in die Pedikelschrauben 12 der Pedikel eines Pedikelpaares eingeschraubt. Dann wird die Verbindungsstange 92 eingesetzt und die oberen Hälften 82 der Lagerelemente 86 an einem an der Gewindebohrung 91 ansetzenden Einführwerkzeug (nicht dargestellt) aufgesetzt. Beim Ausrichten der Verbindungsstange 92 bricht die Verklebung der unteren Hälften 84 mit der Ausnehmung 68 auf, so daß diese Ausrichtung wie oben im Zusammenhang mit der ersten Variante beschrieben möglich ist. Dann werden wie bei der ersten Variante die Klemmelemente 72 aufgesetzt und festgeschraubt.

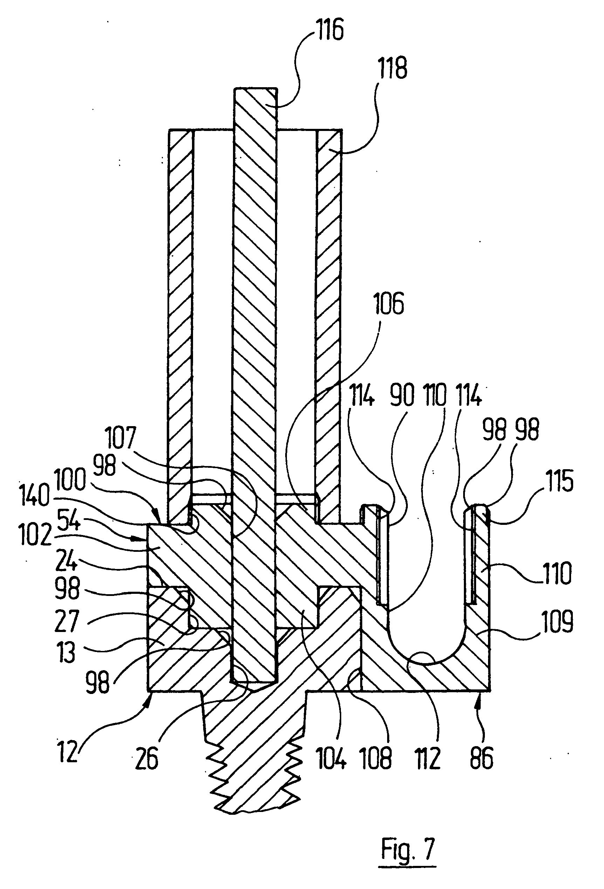

Nun wird unter Bezugnahme auf die Figuren 7 bis 9 ein weiteres Ausführungsbeispiel eines Vergurtungssystems 10 mit einer Verbindungseinrichtung 18 beschrieben, welches jedoch nicht Gegenstand der beanspruchten Erfindung ist. In den Figuren 7 bis 9 tragen Teile, die zu solchen in den Figuren 1 bis 7 funktionsäquivalent sind, die selben Bezugszeichen.

Bei diesem Ausführungsbeispiel trägt die Pedikelschraube 12 einen zylindrischen Kopf 13, in dessen Stirnfläche 24 eine mittige axiale Stufenbohrung vorhanden ist, welche einen kleineren Durchmesser aufweisenden Gewindeabschnitt 26 und einen größeren Durchmesser aufweisenden Führungsabschnitt 27 aufweist. Gewindeabschnitt 26 und Führungsabschnitt 27 weisen an ihrem oberen Rand jeweils eine Einführschräge 98 auf. Die äußere Mantelfläche des Kopfes 13 der Pedikelschraube 12 ist als Außensechskant ausgeführt. Die Stirnfläche 24 ist mit einer strahlenförmig von der Mittelachse der Pedikelschraube 12 ausgehenden Riffelung (nicht sichtbar) versehen. In einem nicht dargestellten weiteren Ausführungsbeispiel ist der Außenmantel des Kopfes 13 der Pedikelschraube 12 ebenfalls mit einer Riffelung versehen.

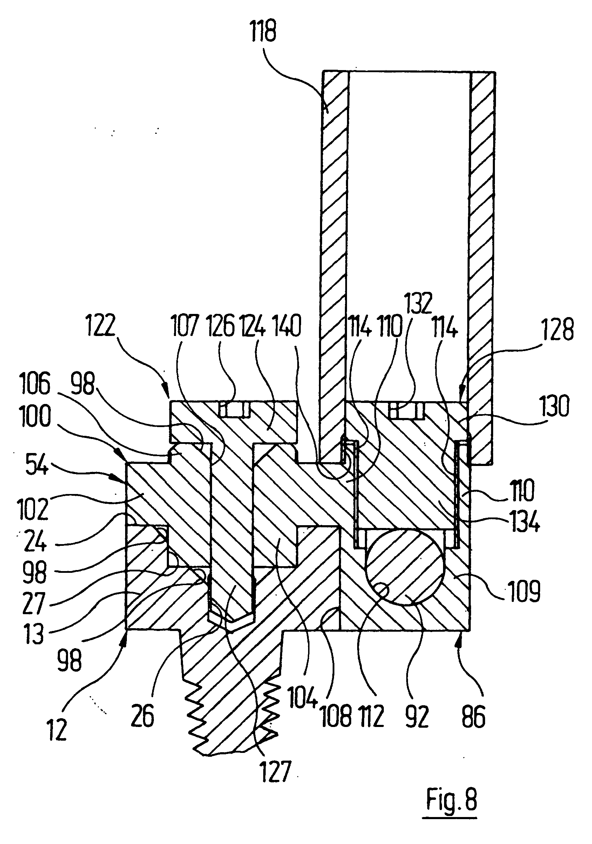

Die Verbindungseinrichtung 18 umfaßt ein in der Draufsicht längliches Befestigungsteil 54 mit halbkreisförmig gerundeten Enden, welches einen Befestigungsabschnitt 100 und einen mit diesem einstückigen Lagerabschnitt 86 aufweist. In einem nicht dargestellten Ausführungsbeispiel ist der Lagerabschnitt gegenüber dem Befestigungsabschnitt um eine Achse verschwenkbar, welche in Einbaulage im wesentlichen senkrecht auf der Längsachse der Pedikelschraube steht. In einer gewünschten Winkelposition ist der Lagerabschnitt dann gegenüber dem Befestigungsabschnitt festlegbar. Der Befestigungsabschnitt 100 wird von einer Durchgangsbohrung 107 durchdrungen, welche in Einbaulage koaxial zur Längsachse der Pedikelschraube 12 liegt. Koaxial zur Durchgangsbohrung 107 weist der Befestigungsabschnitt 100 einen in den Figuren 7 und 8 oberen Gewindeansatz 106 und einen in den Figuren 7 und 8 unteren Führungsvorsprung 104 auf. Der Außendurchmesser und die Höhe des Führungsvorsprungs 104 des Befestigungsabschnitts 100 des Befestigungsteils 54 entsprechen dem Außendurchmesser und der Höhe des Führungsabschnitts 28 im Kopf 13 der Pedikelschraube 12.

Der Durchmesser der Durchgangsbohrung 107 entspricht dem Durchmesser des Gewindeabschnitts 26 der Stufenbohrung im Kopf 13 der Pedikelschraube 12. Die in Einbaulage der Stirnfläche 24 des Kopfes 13 der Pedikelschraube 12 gegenüberliegende Fläche des Befestigungsabschnitts 100 des Befestigungsteils 54 weist ein zu der dort vorhandenen Riffelung komplementäre, in der Zeichnung jedoch nicht sichtbare Riffelung auf.

Der Lagerabschnitt 86 ist seitlich vom Befestigungsabschnitt 100 angeordnet. Er weist eine Gewindebohrung 114 auf, deren Längsachse parallel zur Durchgangsbohrung 107 verläuft. In den Figuren 7 und 8 von oben ist in den Lagerabschnitt 86 mittig durch die Gewindebohrung 114 eine im Querschnitt U-förmige Ausnehmung 90 eingebracht, die der Aufnahme der Verbindungsstange 92 dient und deren Weite daher dem Durchmesser der Verbindungsstange 92 entspricht.

Die Mittelebene der Ausnehmung 90 steht senkrecht auf der durch die Längsachse der Durchgangsbohrung 107 im Befestigungsabschnitt 100 und die Längsachse der Gewindebohrung 114 im Lagerabschnitt 86 aufgespannten Ebene. Die U-förmige Ausnehmung 90 wird durch zwei Seitenwände 110 und einen zylindrisch gekrümmten Bodenabschnitt 112 begrenzt. Die U-förmige Ausnehmung 90 ist tiefer als die Gewindebohrung 114, derart, daß die Gewindebohrung 114 sich vom oberen Rand der U-förmigen Ausnehmung 90 bis nicht ganz auf jene Höhe erstreckt, in der die Seitenwände 110 in den Bodenabschnitt 112 übergehen.

An seinem in den Figuren 7 und 8 oberen Rand trägt der Lagerabschnitt 86 einen zur Gewindebohrung 114 koaxialen Gewindeansatz 115, welcher das gleiche Gewinde wie der Gewindeansatz 106 des Befestigungsabschnitts 100 trägt. In den Figuren 7 und 8 nach unten gesehen steht der Lagerabschnitt 86 über den Führungsvorsprung 104 des Befestigungsabschnitts 100 so über, daß er in Einbaulage in etwa auf Höhe des unteren Randes des Kopfs 13 der Pedikelschraube 12 endet. In seinem in den Figuren 7 und 8 unteren Bereich weist der Lagerabschnitt 86 eine in Einbaulage mit einer Fläche des Außensechskants auf der äußeren Mantelfläche des Kopfes 13 der Pedikelschraube zusammenarbeitende seitliche Anlagefläche 108 auf.

In den Figuren 7 und 8 ist auch eine Führungsstange 116 dargestellt, die an ihrem in Figur 7 unteren Ende ein zum Gewindeabschnitt 26 der Stufenbohrung im Kopf 13 der Pedikelschraube 12 komplementäres Gewinde trägt und in Figur 7 in diesen Gewindeabschnitt 26 eingeschraubt ist. Eine in den Figuren 7 und 8 außerdem gezeigte Führungshülse 118 hat einen Innendurchmesser, welcher dem Außendurchmesser des Gewindeansatzes 106 des Befestigungsabschnitts 100 und des Gewindeansatzes 115 des Lagerabschnitts 86 entspricht. In der Innenwand der Führungshülse 118 ist an deren unterem Ende ein Gewinde 140 vorhanden, welches komplementär zum Gewinde an den Gewindeansätzen 106 bzw. 115 ist.

In Figur 8 sind schließlich noch eine Verbindungsschraube 122 und eine Klemmschraube 128 erkennbar. Die Verbindungsschraube 122 hat einen Kopf 124 mit kreisförmiger Außenkontur und einem Außendurchmesser, welcher unter Gleitspiel dem Innendurchmesser der Führungshülse 118 entspricht. Im Kopf 124 der Verbindungsschraube 122 ist eine Innensechskantausnehmung 126 vorhanden. Der Schraubabschnitt 127 der Verbindungsschraube 122 trägt ein zum Gewindeabschnitt 26 der Stufenbohrung im Kopf 13 der Pedikelschraube 12 komplementäres Gewinde und hat eine Länge, welche geringfügig kleiner ist als die Längserstreckung des Befestigungsabschnitts 100 vom oberen Rand des Gewindeansatzes 106 bis zum unteren Rand des Führungsvorsprungs 104 plus der Tiefe des Gewindeabschnitts 26 im Kopf 13 der Pedikelschraube 12.

Die Klemmschraube 128 verfügt über einen Kopf 130 mit einer Innensechskantausnehmung 132, der mit demjenigen der Verbindungsschraube 122 übereinstimmt. Der Schraubabschnitt 134 der Klemmschraube 128 trägt ein zu der Gewindebohrung 114 im Lagerabschnitt 86 komplementäres Gewinde.

Das Vergurtungssystem 10 kann folgendermaßen eingesetzt werden:

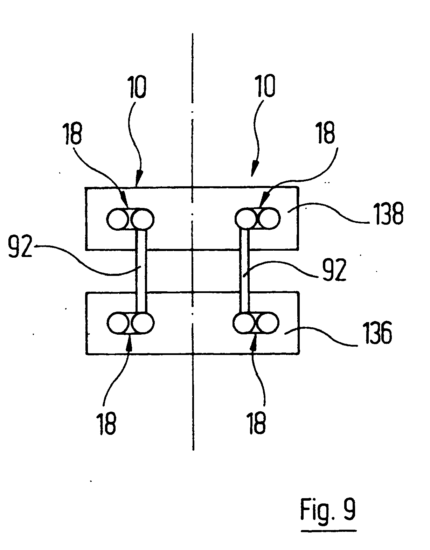

Die Vorbereitung des Operationsgebiets erfolgt wie bereits oben dargelegt. Die grundsätzlichen Verhältnisse im Operationsgebiet sind in Figur 9 grobschematisch dargestellt. Zwei zu verbindende Wirbelkörper tragen dort die Bezugszeichen 136 und 138. Die Wirbelkörper 136 und 138 werden jeweils an zwei Stellen miteinander durch jeweils ein Vergurtungssystem 10 verbunden. Die Positionen im unteren Wirbelkörper 136 werden nachfolgend mit "links unten" und "rechts unten" bezeichnet, die im oberen Wirbelkörper 138 mit "links oben" und "rechts oben".

Zunächst werden mit Hilfe eines üblichen und bekannten Navigationssystems (nicht dargestellt) die exakten Positionen und Ausrichtungen der beiden Pedikelschrauben 12 an den Positionen links unten und links oben bestimmt. Die von diesem Navigationssystem ermittelten Daten werden nun an ein externes in der Zeichnung nicht dargestelltes Modell übertragen.

Bei diesem handelt es sich um eine Vorrichtung, in der die Relativpositionen zweier Pedikelschrauben mit zugehörigen Verbindungseinrichtungen zueinander simuliert werden können, indem eine (ggf. simulierte) Pedikelschraube in entsprechenden Führungen durch Mikrometerschrauben entlang der drei Raumachsen translatorisch und winkelmäßig verstellt und so in eine dem tatsächlichen Operationsgebiet entsprechende Relativposition gegenüber der anderen Pedikelschraube gebracht werden kann. Eine Verbindungsstange wird nun mittels einer entsprechenden Vorrichtung so gebogen, daß sie in die Verbindungseinrichtungen der im Modell befindlichen Pedikelschrauben und damit auch in diejenigen der im Patienten gesetzten Pedikelschrauben paßt.

Auf diese Weise wird also zunächst eine Verbindungsstange 92 entsprechend den tatsächlich auf der linken Seite des Operationsgebietes vorhandenen Gegebenheiten zurechtgebogen.

In den Gewindeabschnitt 26 der Stufenbohrung im Kopf 13 der linken oberen Pedikelschraube 12 wird nun eine Führungsstange 116 eingeschraubt. Auf diese wird dann ein Befestigungsteil 54 einer Verbindungseinrichtung 18 mit Hilfe der im Befestigungsabschnitt 100 vorhandenen Durchgangsbohrung 107 aufgefädelt und bis zum Kopf 13 der Pedikelschraube 12 abgesenkt. Dabei greift der Führungsvorsprung 104 des Befestigungsabschnitts 100 des Befestigungsteils 54 in den Führungsabschnitt 27 der Stufenbohrung im Kopf 13 der Pedikelschraube 12 ein, was durch die am oberen Rand der Stufenbohrung im Kopf 13 der Pedikelschraube 12 vorhandene Einführschräge 98 erleichtert wird.

Die seitliche Anlagefläche 108 am Lagerabschnitt 86 kommt dabei in Anlage an eine Fläche des Außensechskants am Kopf 13 der Pedikelschraube 12, wodurch deren Relativ-Winkelposition festliegt. Eine zusätzliche Sicherung bieten die am Kopf 13 der Pedikelschraube 12 und am Befestigungsabschnitt 100 des Befestigungsteils 54 vorhandenen komplementären Riffelungen. Nun wird über die Führungsstange 116 eine Führungshülse 118 aufgeschoben und mit ihrem Gewinde 920 mit dem Gewindeansatz 106 des Befestigungsabschnitts 100 des Befestigungsteils 54 verschraubt. Eine solche Situation ist in Figur 7 dargestellt.

Die Führungsstange 116 wird nun gelöst und herausgezogen, wobei mit der Führungshülse 118 gegengehalten wird. Eine Verbindungsschraube 122 wird in der Führungshülse 118 in Richtung auf das Befestigungsteil 54 abgesenkt. Dadurch, daß der Durchmesser des Kopfs 124 der Verbindungsschraube 122 im Gleitspiel mit dem Innendurchmesser der Führungshülse 118 zusammenarbeitet, wird die Verbindungsschraube 122 während des Absenkens exakt geführt, so daß sichergestellt ist, daß sie durch die Durchgangsbohrung 107 im Befestigungsabschnitt 100 des Befestigungsteils 54 hindurch in den Gewindeabschnitt 26 im Kopf 13 der Pedikelschraube 12 eingreifen kann. Die Verbindungsschraube 122 wird mit einem Sechskantschlüssel, welcher in die Sechskantausnehmung 126 in ihrem Kopf 124 eingreift, lose verschraubt.

Ein Ende der vorgebogenen Verbindungsstange 92 wird nun außerhalb des Körpers des Patienten in die U-förmigen Querschnitt aufweisende Ausnehmung 90 eines zweiten Befestigungsteils 54 so eingeführt, wie es der im Modell simulierten Situation entspricht. In der U-förmigen Ausnehmung 90 wird die Verbindungsstange 116 durch Festschrauben einer Klemmschraube 128 festgelegt. Die so geschaffene Einheit aus Verbindungseinrichtung 18 (Befestigungsteil 54 und Klemmschraube 128) und Verbindungsstange 92 wird jetzt auf eine zuvor in die Pedikelschraube 12 links unten eingeschraubte Führungsstange 116 mit Hilfe der Durchgangsbohrung 107 im Befestigungsabschnitt 100 des Befestigungsteils 32 aufgefädelt.

Wie bereits bei der Position links oben wird auch hier durch die Führungsstange 116 und die mit dieser im Gleitspiel zusammenarbeitende Durchgangsbohrung 107 im Befestigungsabschnitt 100 des Befestigungsteils 54 ein sicheres und einfaches Einführen der Verbindungseinrichtung 18 gewährleistet.

Das Einführen des Führungsvorsprungs 104 des Befestigungsabschnitts 100 des Befestigungsteils 54 in den Führungsabschnitt 27 der Stufenbohrung im Kopf 13 der Pedikelschraube 12 wird auch hier durch die Einführschräge 98 am oberen Rand des Führungsabschnitts 28 des Kopfes 13 der Pedikelschraube 12 erleichtert. Da die Verbindungsstange 92 ja bereits in der notwendigen Weise abgelängt und zurechtgebogen ist, kommt ihr anderes Ende in der U-förmigen Ausnehmung 90 des linken oberen Befestigungsteils 54 zum Liegen.

Nun wird beim linken unteren Befestigungsteil 54 eine Führungshülse 118 auf den Gewindeansatz 106 des Befestigungsabschnitts 100 des Befestigungsteils 54 aufgeschraubt, die Führungsstange 116 entfernt und, wie oben im Zusammenhang mit dem linken oberen Befestigungsteil 54 beschrieben, die Verbindungsschraube 122 eingeführt und lose verschraubt.

Am linken oberen Befestigungsteil 54 wird die Führungshülse 118 vom Gewindeansatz 106 des Befestigungsabschnitts 100 des Befestigungsteils 54 entfernt und auf den Gewindeansatz 115 des Lagerabschnitts 86 aufgeschraubt. Nun wird eine Klemmschraube 128 in die Führungshülse 118 eingeführt, wobei aufgrund des mit der Führungshülse 118 im Gleitspiel zusammenarbeitenden Kopfes 130 der Klemmschraube 128 eine relativ exakte Führung während des Absenkens der Klemmschraube 128 in die Gewindebohrung 114 im Lagerabschnitt 86 gewährleistet ist (s.o.). Darüber hinaus erleichtern die Einführschrägen 30 an den oberen Rändern der Gewindebohrung 114 im Lagerabschnitt 86 das Einführen der Klemmschraube 128.

Über einen Sechskantschlüssel, der in die Sechskantausnehmung 132 im Kopf 130 der Klemmschraube 128 eingreift, wird die Klemmschraube 128 soweit gedreht, bis sie am entsprechenden Ende der Verbindungsstange 92 lose anliegt. Diese Situation ist in Figur 8 dargestellt.

Beide Befestigungsteile 32, also jenes an der linken unteren als auch jenes an der linken oberen Position, sind zum gegenwärtigen Zeitpunkt noch mit einer Führungshülse 118 verbunden, die durch die Hautinzisionen nach außen über die Haut des Patienten übersteht. Mit diesen Führungshülsen 118 kann nun der Operateur, falls erforderlich, noch eine Feinjustierung der Relativposition der beiden zu verbindenden Wirbelkörper 136 und 138 vornehmen. Sobald die endgültige Position erreicht ist, wird einerseits die Verbindungsschraube 122 am linken unteren Befestigungsteil 54 und andererseits die Klemmschraube 128 am linken oberen Befestigungsteil 54 festgezogen. Anschließend werden die Innensechskantschlüssel und die entsprechenden Führungshülsen 118 entfernt. Die beiden Wirbelkörper 136 und 138 sind nun auf ihrer linken Seite starr miteinander verbunden.

Der gleiche Vorgang wiederholt sich nun auf der rechten Seite, wo zunächst jedoch von den dortigen Pedikelschrauben 12 noch das Repositionsinstrument abgenommen werden muß. Schließlich sind die beiden Wirbelkörper 136 und 138 insgesamt über vier Pedikelschrauben 12, vier Verbindungseinrichtungen 116 und zwei Verbindungsstangen 92 miteinander starr verbunden. Dies ist.schematisch in Figur 9 dargestellt.

## Patentansprüche

1. Vergurtungssystem zur Spondylodese der Lendenwirbelsäule mit
a) mindestens zwei jeweils in einem Pedikel verankerbaren Verankerungselementen (12);
b) für jedes Verankerungselement einer diesem zugeordneten Befestigungseinrichtung (26) für ein Repositionsinstrument (20);
c) mindestens eine zwischen benachbarten Wirbelkörpern verlaufenden Verbindungsstange (92); und
d) für jedes Verankerungselement einer an diesem befestigbaren Verbindungseinrichtung (18) für die Verbindungsstange (92);
e) für jedes Verankerungselement (12) einem Verlängerungselement (14), welches mit einem Ende an der Befestigungseinrichtung (26) des Verankerungselement (12) und an dessen anderem Ende das Repositionsinstrument (20) lösbar befestigt werden kann, wobei das Verlängerungselement (14) mit dem Verankerungselement (12) verschraubbar ist,
**dadurch gekennzeichnet, dass**
das Verlängerungselement (14) auch mit dem Repositionsinstrument (20) verschraubbar ist.

2. Vergurtungssystem nach Anspruch 1, **dadurch gekennzeichnet, daß** das Verlängerungselement (14) an dem Verankerungselement (12) axial fluchtend befestigbar ist.

3. Vergurtungssystem nach einem der vorhergehenden Ansprüche, **dadurch gekennzeichnet, daß** das Verlängerungselement (14) einen Angriffsabschnitt (40) aufweist.

4. Vergurtungssystem nach einem der vorhergehenden Ansprüche, **dadurch gekennzeichnet, daß** die Verbindungseinrichtung (18) ein mit dem Verankerungselement (12) lösbar verbindbares Befestigungsteil (54) mit einem zumindest bereichsweise kugelpfannenförmigen Lagerabschnitt (58), ein zweigeteiltes Lagerelement (86) für die Verbindungsstange (92) mit einem mindestens bereichsweise kugelig gekrümmten, zu dem kugelpfannenförmigen Lagerabschnitt (58) komplementären Lagerabschnitt und ein Klemmelement (72) umfaßt, welches die Verbindungsstange (92), das Befestigungsteil (54) und das Lagerelement (86) in Einbaulage beaufschlagt und hierdurch festlegt.

5. Vergurtungssystem nach Anspruch 4, **dadurch gekennzeichnet, daß** die kugelig gekrümmten Abschnitte (86, 102, 123) und die jeweiligen zu ihnen komplementären kugelpfannenförmigen Abschnitte (58, 114, 125) Oberflächen mit einem hohen Reibungswert aufweisen, derart, daß sie sich unter Pressung nicht gegeneinander verschieben können.

## Claims

1. Connection system for the spinal fusion of the lumbar spinal column, comprising
a) at least two anchoring elements (12), each of which can be anchored in a pedicle;
b) for each anchoring element, an associated fastening device (26) for a repositioning instrument (20);
c) at least one connecting rod (92) extending between adjacent vertebrae; and
d) for each anchoring element, a connecting device (18) for the connecting rod (92), which connecting device (18) can be fastened to the anchoring element;
e) for each anchoring element (12), a lengthening element (14) which can be releasably fastened at one end to the fastening device (26) of the anchoring element (12) and to the other end of which the repositioning instrument (20) can be releasably fastened, wherein the lengthening element (14) can be screwed to the anchoring element (12),
**characterised in that**
the lengthening element (14) can also be screwed to the repositioning instrument (20).

2. Connection system according to claim 1, **characterised in that** the lengthening element (14) can be fastened to the anchoring element (12) in axial alignment.

3. Connection system according to any one of the preceding claims, **characterised in that** the lengthening element (14) has an engagement portion (40).

4. Connection system according to any one of the preceding claims, **characterised in that** the connecting device (18) comprises a fastening part (54) which can be releasably connected to the anchoring element (12) and has a bearing portion (58) which is ball-socket-shaped at least in some areas, a two-part bearing element (86) for the connecting rod (92), which bearing element (86) has a bearing portion which at least in some areas is spherically curved and complementary to the ball-socket-shaped bearing portion (58), and a clamping element (72) which acts upon and thereby fixes the connecting rod (92), the fastening part (54) and the bearing element (86) in the fitted position.

5. Connection system according to claim 4, **characterised in that** the spherically curved portions (86, 102, 123) and the respective complementary ball-socket-shaped portions (58, 114, 125) have surfaces with a high coefficient of friction so that they are not able to move relative to one another when pressed.

## Revendications

1. Système de liaison destiné à l'arthrodèse de la région lombaire de la colonne vertébrale, avec
a) au moins deux éléments d'ancrage (12) pouvant être respectivement ancrés dans un pédicule ;
b) pour chaque élément d'ancrage, un dispositif de fixation (26) associé à cet élément et destiné à un instrument de repositionnement (20) ;
c) au moins une tige de jonction (92) s'étendant entre deux corps vertébraux voisins ; et
d) pour chaque élément d'ancrage, un dispositif de jonction (18) pouvant être fixé sur cet élément et destiné à la tige de jonction (92) ;
e) pour chaque élément d'ancrage (12), un élément de prolongement (14) qui peut être fixé de manière amovible par une extrémité au dispositif de fixation (26) de l'élément d'ancrage (12) et à l'autre extrémité duquel l'instrument de repositionnement (20) peut être fixé de manière amovible, sachant que l'élément de prolongement (14) peut être assemblé par vissage à l'élément d'ancrage (12),
**caractérisé en ce que** l'élément de prolongement (14) peut également être assemblé par vissage à l'instrument de repositionnement (20).

2. Système de liaison selon la revendication 1, **caractérisé en ce que** l'élément de prolongement (14) peut être fixé en alignement axial à l'élément d'ancrage (12).

3. Système de liaison selon l'une des revendications précédentes, **caractérisé en ce que** l'élément de prolongement (14) présente une partie de prise (40).

4. Système de liaison selon l'une des revendications précédentes, **caractérisé en ce que** le dispositif de jonction (18) comprend une pièce de fixation (54) pouvant être assemblée de manière amovible à l'élément d'ancrage (12) et pourvue d'une partie de palier (58) au moins sectoriellement en forme de coussinet sphérique, un élément de palier (86) en deux parties, destiné à la tige de jonction (92) et pourvu d'une partie de palier au moins sectoriellement à courbure sphérique, complémentaire de la partie de palier (58) en forme de coussinet sphérique, et un élément de serrage (72) qui, en position de montage, sollicite la tige de jonction (92), la pièce de fixation (54) et l'élément de palier (86) et les immobilise ainsi.

5. Système de liaison selon la revendication 4, **caractérisé en ce que** les parties (86, 102, 123) à courbure sphérique et les parties respectives (58, 114, 125) en forme de coussinet sphérique qui leur sont complémentaires présentent des surfaces ayant un coefficient de friction élevé, de telle sorte qu'elles ne peuvent pas se décaler les unes par rapport aux autres sous l'action d'une pression d'application.
